# EUROPÄISCHE PATENTSCHRIFT

(11) **EP 0 382 807 B1**
(45) Veröffentlichungstag und Bekanntmachung des Hinweises auf die Patenterteilung: **07.12.1994**
(21) Anmeldenummer: 89907089.0
(22) Anmeldetag: 29.06.1989
(51) Int. Cl.: A61B 3/10

(54) **VORRICHTUNG ZUR ERZEUGUNG EINES BILDES EINES OBJEKTS**
DEVICE FOR PRODUCING AN IMAGE OF AN OBJECT
GENERATEUR D'IMAGES D'OBJETS

(30) Priorität: 29.06.1988 DE 3821975; 29.06.1988 DE 3821977
(43) Veröffentlichungstag der Anmeldung: 22.08.1990
(73) Patentinhaber: G. RODENSTOCK INSTRUMENTE GMBH, 85521 Ottobrunn (DE)
(72) Erfinder: TRILLER, Adolf, D-8032 Lochham (DE); KLINGBEIL, Ulrich, D-8000 München 81 (DE); PLESCH, Andreas, D-8000 München 40 (DE)
(74) Vertreter: Münich, Wilhelm, Dr.
(86) Internationale Anmeldenummer: DE8900433
(87) Internationale Veröffentlichungsnummer: WO9000025

(56) Entgegenhaltungen:
- EP-A- 0 167 877
- WO-A-88/03396
- US-A- 4 370 034
- PATENT ABSTRACTS OF JAPAN, Band 9, Nr. 137 (P-363)(1860), 12. Juni 1985 ; & JP-A-60 017 716

## Beschreibung

### Technisches Gebiet

Die Erfindung bezieht sich auf eine Vorrichtung zur Erzeugung eines Bildes eines Objekts und insbesondere zur Beobachtung des Auges gemäß dem Oberbegriff des Patentanspruchs 1.

### Stand der Technik

Bei der Beobachtung der hinteren Augenabschnitte besteht die Schwierigkeit, daß die Beleuchtung und die Beobachtung durch die Augenpupille und die häufig optisch nicht klaren vorderen Augenmedien erfolgen muß, an denen Reflexe auftreten, und die Abbildungsfehler erzeugen.

Es ist deshalb bereits seit längerem vorgeschlagen worden, zur Beobachtung der hinteren Augenabschnitte anstelle von konventionellen Funduskameras scannende bzw. abtastende Vorrichtungen zu verwenden, bei denen der Augenhintergrund nicht großflächig ausgeleuchtet wird, sondern die mit einem auf einem möglichst kleinen Fleck fokussierten Beleuchtungslichtstrahl den Augenhintergrund abtasten und das reflektierte Licht in Zuordnung zur Abtastsequenz erfassen. Hierzu wird beispielsweise auf "The Foundations of Ophtalmology, Band 7, S. 307/308, Jahrgang 1962, die US-PS 4 213 678, die japanischen Patentveröffentlichungen JP-B-61-5730 und JP-A-50-138822 sowie die EP-A-0 145 563 verwiesen.

Die aus den vorstehend genannte Druckschriften bekannten Vorrichtungen ermöglichen eine Übersichtsaufnahme des Augenhintergrundes. Es wäre jedoch - wie erfindungsgemäß erkannt worden ist - gerade aufgrund der hervorragenden Qualität der von abtastenden Vorrichtungen gelieferten Bilder insbesondere bei der Untersuchung der hinteren Augenabschnitte vorteilhaft, wenn der Bildausschnitt geändert und Details vergrößert werden könnten.

### Darstellung der Erfindung

Der Erfindung liegt deshalb die Aufgabe zugrunde eine Vorrichtung zur Erzeugung eines Bildes eines Objekts und insbesondere zur Beobachtung des Auges anzugeben, bei der eine Änderung der Größe des beobachteten Abschnitts und insbesondere eine Vergrößerung bestimmter Bereiche möglich ist.

Eine erfindungsgemäße Lösung dieser Aufgabe ist mit Ihren Weiterbildungen in den Patentansprüchen angegeben.

Erfindungsgemäß wird eine Anordung aus zwei Linsen oder Spiegeln vorgeschlagen, die im Strahlengang zwischen den strahlablenkenden Elementen, also beispielsweise zwischen einem Polygonspiegel und einem Schwing- bzw. Galvanometerspiegel angeordnet ist, und die "als Einheit" zur Änderung des horizontalen Ablenkwinkels austauschbar ist.

Dabei ist es besonders bevorzugt, wenn gemäß Anspruch 3 die beiden optischen Elemente eine afokale Anordnung bilden.

Die Abstände der optischen Elemente zueinander sowie zu den Horizontal- und Vertikal-Ablenkelementen können bevorzugt gemäß Anspruch 5 so gewählt sein, daß beim Austausch der optischen Elemente der Abbildungsmaßstab (von Horizontal- auf Vertikal-Ablenkelementen) den reziproken Wert des ursprünglichen Vergrößerungsmaßstabes annimmt, da dann keine Anderung der optischen Weglänge auftritt, die eine Verschiebung des "Spiegelortes" beim Austauschen nötig machen würde. Selbstverständlich ist es aber auch möglich, andere Maßstäbe zu wählen, und die auf den Wechsler angebrachten optischen Elemente so auszubilden, daß beim Wechseln die geänderte optische Weglänge berücksichtigt wird.

Da der Vergrößerungswechsler an einer Stelle im Strahlengang angeordnet ist, am der der abtastende Beleuchtungslichtstrahl nur in einer Richtung, nämlich horizontal aufgefächert ist, können die Abmessungen der optischen Elemente klein sein. Dies ist besonders bei Spiegelsystemen (Anspruch 2) konstruktiv von Vorteil; gleichzeitig sind die nötigen Kippwinkel der Spiegel und damit die Abbildungsfehler wesentlich kleiner als bei einer Anordnung der Vergrößerungswechslers an einer anderen Stelle des optischen Gesamtsystems, nämlich zwischen Abtasteinrichtung und Auge.

Durch die im Anspruch 2 beanspruchte Verwendung von Spiegeln erhät man gegenüber der Verwendung von Linsen den Vorteil, daß das System praktisch reflexfrei und achromatisch ist.

Die geringen Restabbildungsfehler der Vergrößerungswechslers können durch den Einsatz von Mangin-Spiegeln anstelle von Oberflächenspiegeln weiter reduziert werden (Anspruch 4).

Die vorstehend beschriebenen erfindungsgemäßen Maßnahmen bewirken eine Veränderung des Abbildungsmaßstabes in Horizontalrichtung; selbstverständlich kann durch ähnliche Maßnahmen auch der Abbildungsmaßstab in Vertikalrichtung "optisch geändert" werden.

Besonders bevorzugt ist es jedoch, wenn nach Anspruch 6 die Änderung der Vertikalvergrößerung zur Anpassung an die durch den Austausch von Elementen geänderte Horizontalvergrößerung dadurch erfolgt, daß die Vertikal-Ablenkeinrichtung, also beispielsweise ein Galvanometerspiegel, entsprechend angesteuert wird. Dies kann in einfacher Weise elektronisch erfolgen.

Die erfindungsgemäße Möglichkeit, eine Änderung der Größe des beobachteten Abschnitts und insbesondere eine Vergrößerung bestimmter Bereiche vorzunehmen, läßt insbesondere bei der Beobachtung der hinteren Augemabschnitte eine Scharfeinstellung beispielsweise zur Refraktionskompensation als wünschenswert erscheinen.

Ein Vorschlag, eine derartige Scharfeinstellung zu realisieren, ist zwar bereits aus der EP-A-0 145 563 bekannt. Bei dieser Vorrichtung werden sowohl der Beleuchungs- als auch der Beobachtungslichtstrahl über die Abtasteinrichtung geführt. Die Teilung zwischen Beobachtungs- und Beleuchtungslichtstrahl erfolgt dabei unmittelbar hinter (in Richtung des reflektierten Lichts betrachtet) bzw. vor (in Richtung des Beleuchtungslichts betrachtet) der Abtasteinrichtung. In dem Teil des Lichtwegs, in dem der Beobachtungs- und der Beleuchtungslichtstrahl getrennt sind, sind Einrichtungen zur Refraktionskompensation vorgesehen, die zur Refraktionskompensation synchron bewegt werden.

Diese bekannte Einrichtung zur Beobachtung des Auges hat jedoch eine Reihe von Nachteilen:
Zum einen ist es erforderlich, Elemente vorzusehen, die die in den jeweiligen Strahlengängen vorgesehenen optischen Bauteile zur Refraktionskompensation synchron bewegen. Zum anderen ist der Justieraufwand sehr hoch, da zur Refraktionskompensation verhältnismäßig viele Elemente vorgesehen werden müssen. Desweiteren ist es bei dieser bekannten Einrichtung praktisch nicht möglich, die optische Weglänge zu verändern.

Im Anspruch 7 ist eine Weiterbildung einer erfindungsgemäßen Vorrichtung angegeben, die eine Refraktionskompensation mit guten optischen Eigenschaften und einem vergleichsweise geringen technischen Aufwand ermöglicht. Diese erfindungsgemäße Lösung kann selbstverständlich auch bei einer gattungsgemäßen Vorrichtung, d.h. bei einer Vorrichtung ohne Vergrößerungsumschaltung verwendet werden.

Erfindungsgemäß ist erkannt worden, daß es nicht nur möglich, sondern auch besonders vorteilhaft ist, die Elemente zur Scharfeinstellung bzw. Refraktionskompensation in dem Teil des Lichtwegs anzuordnen, der vom Beleuchtungs- und Beobachtungslichtstrahl gemeinsam durchlaufen wird. Es ist jedoch - wie ebenfalls erfindungsgemäß erkannt worden ist - nicht sinnvoll, die Einrichtung zur Refraktionskompensation im Lichtweg der Abtasteinrichtung oder im Lichtweg zwischen Abtasteinrichtung und Auge anzuordnen, da dann aufgrund der Auffächerung des Lichtstrahls vergleichsweise große optische Elemente erforderlich wären.

Deshalb ist erfindungsgemäß die Einrichtung zur Refraktionskompensation zwischen der Abtasteinrichtung und dem optischen Element angeordnet, durch das der Beleuchtungs- und der Beobachtungslichtweg getrennt werden. Dieses Element kann beispielsweise ein Teilerspiegel sein.

Um den für die Einrichtung zur Refraktionskompensation benötigten Platz zu schaffen, wird die Pupillenebene mittels eines "Relais-Systems" zwischenabgebildet. Diese Zwischenabbildung wird durch eine Anordnung von mindestens zwei Linsen und/oder Spiegeln bewirkt; ferner können zur Kompensation des Lichtwegs nicht abbildende Spiegel (Anspruch 11) vorgesehen werden, die den Lichtweg umlenken und zur Wegänderung gemeinsam verschoben werden.

Damit ist es möglich, durch den Austausch bzw. das aus dem Strahlengang "Herausnehmen" (Anspruch 12) eines abbildenden optischen Elements, wie einer Linse (Anspruch 10), eines Linsensystems und/oder durch die Verschiebung eines abbildenden optischen Elements eine Änderung der Divergenz des Beleuchtungs-und Beobachtungslichtstrahls herbeizuführen, durch die unterschiedliche Refraktionen beispielsweise der zu untersuchenden Augen ausgeglichen werden. Zusätzlich kann mittels dieser Elemente auf verschiedene Ebenen innerhalb des Auges scharf eingestellt werden.

Darüberhinaus hat die erfindungsgemäße Vorrichtung gegenüber einem Vorschlag von Webb den Vorteil, daß die Pupillenebene direkt auf das scannende Element der Abtasteinrichtung, also beispielsweise einen Polygon- oder Schwingspiegel gelegt werden kann, so daß sich ein symmetrischer Strahlengang ergibt.

In jedem Falle hat die erfindungsgemäße Vorrichtung den Vorteil, daß die Refraktionskompensation konstruktiv und vom Justieraufwand deutlich einfacher als beim Stand der Technik ausgeführt ist.

### Kurze Beschreibung der Zeichnung

Die Erfindung wird nachstehend anhand eines Ausführungsbeispiels unter Bezugnahme auf die Zeichnung naher beschrieben, in der zeigen:
- Fig. 1: die erfindungsgemäße Vergrößerungsumschaltung, und
- Fig. 2: die erfindungsgemäße Scharfeinstellung.

### Beschreibung von Ausführungsbeispielen

Die erfindungsgemäße Vorrichtung weist eine in den Figuren nicht dargestellte Beleuchtungs-Lichtquelle, beispielsweise einen Laser, sowie eine nicht dargestellte Detektoreinrichtung auf, deren Ausgangssignal von einer Auswerte- und Synchronisiereinheit ausgewertet und beispielsweise auf einem Monitor dargestellt wird. Bei dem gezeigten Ausführungsbeispiel "verlaufen" sowohl der Beleuchtungslichtstrahl 14 als auch der vom Augenhintergrund kommende Lichtstrahl 15 über die Ablenkeinrichtung.

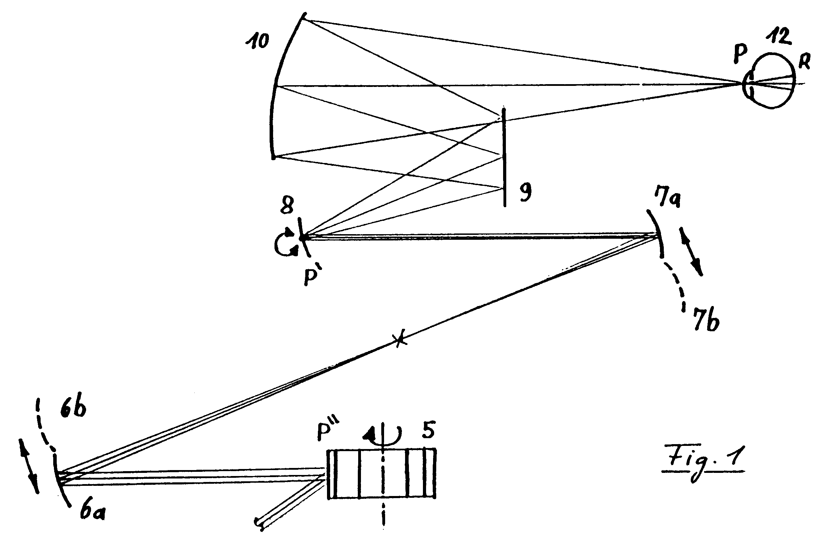

Fig. 1 zeigt, daß der Lichtstrahl 14 des Lasers von dem Horizontal-Scanner, der bei dem gezeigten Ausführungsbeispiel ein sich drehender Polygonspiegel 5 ist, in Horizontalrichtung (senkrecht zur Zeichenebene) abgelenkt wird. Der nun in der Horizontalebene aufgefächerte Strahl durchläuft das Spiegelsystem 6 und 7, und trifft auf einen Vertikal-Scanner, der bei dem gezeigten Ausführungsbeispiel ein Schwing- bzw. Galvanometerspiegel 8 ist, auf. Nach dem Spiegel 8 hat das Strahlbündel einen "rechteckigen" Querschnitt. Nach Umlenkung an einem Planspiegel 9 wird er von einem Konkavspiegel 10 auf das zu untersuchende Auge 12 abgebildet. Der reflektierte Lichtstrahl 15 durchläuft in umgekehrter Reihenfolge die genannten Elemente und wird nach dem Horizontal-Ablenkelement 5 von einem nicht dargestellten Detektor nach vorheriger Trennung des Beleuchtungs- und des Beobachtungslichtwegs nachgewiesen.

Bei dem gezeigten Ausführungsbeispiel können zur Änderung der Vergrößerung die Elemente 6a und 7a paarweise durch die Elemente 6b und 7b ersetzt werden. Die Elemente 6a und 7a sowie die Elemente 6b und 7b bilden dabei jeweils ein afokales System, deren Vergrößerungsmaßstähe bevorzugt zueinander reziprok sind.

Auch der Schwingspiegel 8 und der abbildende Spiegel 10 bilden (gemeinsam mit dem Spiegel 9) ein afokales System.

Synchron mit der Horizontalvergrößerung muß die Vertikalvergrößerung geändert werden. Dies kann durch eine elektronisch ansteuerbare Ablenkeinrichtung, z.B. einen Galvanometerscanner realisiert werden.

Die erfindungsgemäße Vorrichtung ermöglicht es damit, die Größe des beobachteten Bereichs (also beispielsweise des betrachteten Bereichs des Augenhintergrunds) zu ändern, d.h. die Vergrößerung des Gesamtsystems umzuschalten.

Durch die Kombination von zwei Spiegeln als abbildende und Vergrößerungs-bestimmende Elemente ergeben sich eine Reihe von Vorteilen, wie geringe Abbildungsfehler. Reflexfreiheit, Achromazität sowie durch die Faltung des Strahlenganges ein geringer Platzbedarf.

Dabei ist es besonders bevorzugt, daß beim Vertauschen der Spiegel 6a und 6b bzw. 7a und 7b der Abbildungsmaßstab zwischen dem horizontal ablenkenden Element 5 und dem vertikal ablenkemden Element 11 den reziproken Wert annimmt, da dann keine Änderung der optischen Weglänge eintritt und die Spiegel 6a und 6b bzw. 7a und 7b nur ausgetauscht, nicht jedoch zur Kompensation der optischen Weglänge verschoben werden müssen.

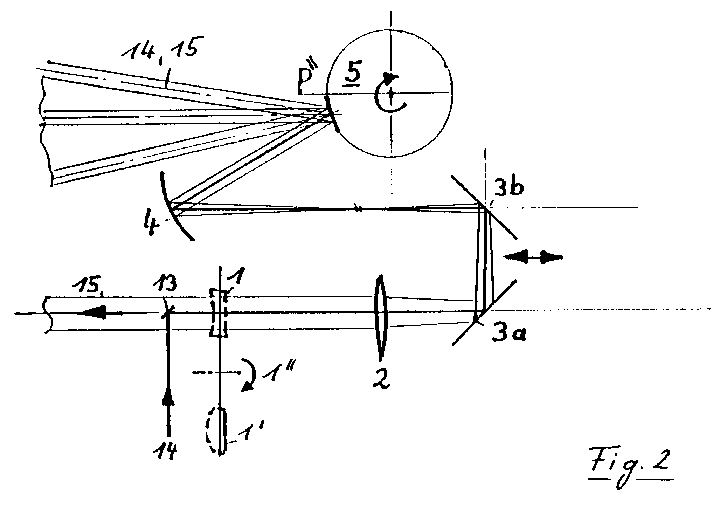

Fig. 2 zeigt den Teil der erfindungsgemäßen Vorrichtung, in dem die Scharfeinstellung und insbesondere die Refraktionskompensation erfolgt. Ein Teilerspiegel 13 trennt den Beleuchtungslichtweg 14 und den Beobachtungslichtweg 15. Bei dem gezeigten Ausführungsbeispiel ist das trennende optische Element 13 ein kleiner Spiegel, der zu einer sogenannten invertierten Gullstrand-Pupille führt, wie sie in der US-PS 4 213 678 vorgeschlagen worden ist.

Es ist selbstverständlich aber auch möglich, eine normale "Gullstrand-Pupille", wie sie in der japanischen Patentveröffentlichung JP-B-61-5730 verwendet wird, nebeneinander liegende Pupillen, wie sie in der japanischen Patentveröffentlichung JP-A-50-138822 vorgeschlagen werden oder einander überlagernde Pupillen, wie sie der EP-A-0 145 563 entnehmbar sind, zu verwenden.

Zwischen dem Teilerspiegel 13 und dem Polygonspiegel 5 der Abtasteinrichtung ist die erfindungsgemäß aufgebaute Einrichtung zur Scharfeinstellung bzw. zur Verschiebung der Schärfenebene sowie zur Refraktionskompensation bzw. zur Scharfeinstellung auf verschiedene Ebenen des untersuchten Objekts vorgesehen.

Diese Einrichtung weist austauschbare Linsen 1 bzw. 1', die beispielsweise auf einem Revolver 1'' angeordnet sind, eine feststehende Linse 2, zwei gemeinsam in Richtung des Pfeils verschiebbare Planspiegel 3a und 3b sowie einen Konkavspiegel 4 auf. Die Elemente 2 und 4 bewirken eine Zwischenabbildung der Pupillenebene P'', die bei der erfindungsgemäßen Vorrichtung direkt auf die Spiegelfläche des Polygonspiegels 5 gelegt ist.

Für das rechtsichtige Auge bilden Linse 2 und Hohlspiegel 4 ein afokales System. Bei Fehlsichtigkeit wird eine entsprechende Linse 1 des Revolvers bzw. Linsenrades 1'' vorgeschaltet und zum Feinabgleich die Umlenkspiegel 3a und 3b verschoben, so daß der austretende Strahl parallel verläuft. Anders ausgedrückt wird durch den Austausch (bzw. das Weglassen) der Linse 1 die Divergenz des Strahlengangs leicht geändert, so daß unterschiedliche Augenrefraktionen grob kompensierbar sind. Gleichzeitig wird durch Verschieben der Spiegel 3a und 3b die Länge des Lichtwegs verändert und eine Feineinstellung durchgeführt.

Ferner ist es möglich, durch die erfindungsgemäße Einrichtung zur Scharfeinstellung nicht nur unterschiedliche Augenrefraktionen zu kompensieren, so daß der Augenhintergrund unabhängig von eventuellen Fehlsichtigkeiten immer scharf beobachtet werden kann, sondern auch eine Verschiebung der Schärfenebene durchzuführen.

Eine derartige Verschiebung erlaubt insbesondere dann, wenn eine zu einer geringen Schärftentiefe führende Pupillenteilung zwischen Beleuchtungs- und Beobachtungslicht gewählt wird, die Beobachtung und die Aufnahme von verschiedenen "Schnittebenen".

Vorstehend ist die Erfindung anhand eines Ausführungsbeispiels ohne Beschränkung des allgemeinen Erfindungsgedankens - bei einer bildgebenden Scannvorrichtung durch den Austausch vom zwei einander zugeordneten abbildenden Elementen den Horizontal-Abbildungsmaßstab zu ändern sowie gegebenenfalls den Vertikal-Abbildungsmaßstab durch entsprechende Ansteuerung des Vertikal-Ablenkelements anzupassen - beschrieben worden. Innerhalb dieses allgemeinen Erfindungsgedankens sind selbstverständlich die verschiedensten Modifikationen möglich:
So können auch andere optische Elemente, wie Linsen oder dgl. verwendet werden. Selbstverständlich kann die beschriebene erfindungsgemäße Vorrichtung nicht nur zur Beobachtung des Augenhintergrundes, sondern auch zu anderen Zwecken eingesetzt werden, zu denen ansonsten bildgebende Scanvorrichtungen verwendet werden. Bei der Beobachtung des Augenhintergrundes ist ihr Einsatz jedoch besonders bevorzugt.

Weiterhin ist es selbstverständlich möglich, anstelle von austausch-barem optischen Elementen 1 auch verschiebbare optische Elemente vorzusehen, so daß eine kontinuierliche Einstellung möglich wird. Ferner können anstelle von Spiegeln und Linsen als abbildende optische Elemente lediglich Spiegel oder lediglich Linsen eingesetzt werden.

Die gezeigte Anordnung hat jedoch den Vorteil, daß sie zu einer Faltung des Lichtwegs führt, durch die ein platzsparender Aufbau der erfindungsgemäßen Vorrichtung möglich wird.

Ferner kann die erfindungsgemäße Einrichtung nicht nur bei Laser-Scanning-Ophthalmoskopen, sondern auch bei beliebigen Einrichtungen zur Gewinnung eines Bildes für beliebige Anwendungszwecke zur Scharfeinstellung und/oder zur Einstellung der Schärfenebene eingesetzt werden.

## Patentansprüche

1. Vorrichtung zur Erzeugung eines Bildes eines Objekts und insbesondere zur Beobachtung der hinteren Abschnitte eines Auges mit einer Beleuchtungslichtquelle, deren Licht auf dem Objekt fokussierbar ist,
einer Abtasteinrichtung (5,8), die eine Abtastbewegung des Lichtstrahls der Beleuchtungslichtquelle über dem Objekt erzeugt und strahlenablenkende sowie abbildende optische Elemente aufweist,
einer Detektoreinrichtung, die das an dem Objekt reflektierte Licht empfängt, und
einer Auswerte- und Synchronisiereinheit, die aus dem zeitsequentiellen Ausgangssignal der Detektoreinrichtung ein Bild des Objekts erzeugt,
dadurch **gekennzeichnet**, daß zur Änderung des horizontalen Ablenkwinkels eine Anordnung aus mindestens zwei abbildenden optischen Elementen (6a,6b,7a,7b) sowie Mittel zum Einbringen jeweils eines dieser abbildenden optischen Elemente in den Strahlengang zwischen den strahlablenkenden Elementen (5,8) vorgesehen sind.

2. Vorrichtung nach Anspruch 1,
dadurch **gekennzeichnet**, daß die abbildenden optischen Elemente Spiegel sind.

3. Vorrichtung nach Anspruch 1 oder 2,
dadurch **gekennzeichnet**, daß die Anordnung zwei Spiegel (6a,6b,7a,7b) oder Linsen aufweist, die ein afokales System bilden.

4. Vorrichtung nach Anspruch 3,
dadurch **gekennzeichnet**, daß die abbildenden optischen Elemente Manginspiegel sind.

5. Vorrichtung nach einem der Ansprüche 1 bis 4,
dadurch **gekennzeichnet**, daß zwei Sätze von abbildenden optischen Elementen (6a,6b,7a,7b) vorgesehen sind, die mittels eines Vergrößerungswechslers gegeneinander austauschbar sind, und deren Vergrößerungsmaßstäbe zueinander reziprok sind.

6. Vorrichtung nach einem der Ansprüche 1 bis 5,
dadurch **gekennzeichnet**, daß zur Änderung der Vertikalvergrößerung eine Vertikal-Ablenkeinrichtung (8) entsprechend ansteuerbar ist.

7. Vorrichtung nach einem der Ansprüche 1 bis 6,
dadurch **gekennzeichnet**, daß zur Scharfeinstellung bzw. zur Verschiebung der Schärfenebene und zur Refraktionskompensation zwischen einem Auskoppelelement (13), das das Beleuchtungslicht (14) und das reflektierte Licht (15) trennt, und der Abtasteinrichtung (5) ein optisches System (1,2,4) vorgesehen ist, das eine zur Pupille des zu untersuchenden Auges konjugierte Pupillenebene P'' zwischenabbildet, und das wenigstens eine austauschbare und/oder verschiebbare optische Komponente (1) enthält.

8. Vorrichtung nach Anspruch 7,
dadurch **gekennzeichnet**, daß das optische System zwei stationär angeordnete abbildende optische Elemente (2,4) aufweist.

9. Vorrichtung nach Anspruch 8,
dadurch **gekennzeichnet**, daß die stationären optischen Elemente (2,4) ein afokales System bilden.

10. Vorrichtung nach einem der Ansprüche 7 bis 9,
dadurch **gekennzeichnet**, daß im Strahlengang zwischen Abtastsystem (5) und Auskoppelelement (13) auf einem Linsenrad (1'') eine Linse (1) austauschbar angeordnet ist.

11. Vorrichtung nach einem der Ansprüche 7 bis 10,
dadurch **gekennzeichnet**, daß das optische System zusätzlich wenigstens zwei Spiegel (3a,3b) aufweist, die den Strahlengang in einer Ebene um jeweils 90° umlenken und zur Veränderung der optischen Weglänge gemeinsam verschiebbar sind.

12. Vorrichtung nach einem der Ansprüche 7 bis 11,
dadurch **gekennzeichnet**, daß das Linsenrad (1'') eine Stellung aufweist, in der keine zusätzliche Linse (1) in den Strahlengang einbringbar ist

## Claims

1. Device for producing an image of an object, more particularly for observation of the rear portions of an eye, with a source of illumination whose light may be focused on the object, with a scanning device (5, 8) producing a scanning movement of the beam from the source of illumination over the object, and having beam-deflecting and imaging optical elements, with a detector, device which receives light reflected from the object, and with an evaluation and synchronising unit which produces from the time-sequential output signal of the detector device an image of the object, characterised in that there is provided, in order to alter the horizontal angle of deflection, an arrangement comprising at least two imaging optical elements (6a, 6b, 7a, 7b), and means of respectively bringing one of these imaging optical elements into the beam path between the beam-deflecting elements (5, 8).

2. Device according to claim 1, characterized in that the imaging optical elements are mirrors.

3. Device according to claim 1 or 2, characterized in that the arrangement has two mirrors (6a, 6b, 7a, 7b) or lenses, which form an afocal system.

4. Device according to claim 3, characterized in that the imaging optical elements are Mangin mirrors.

5. Device according to one of claims 1 to 4, characterized in that two sets of imaging optical elements (6a, 6b, 7a, 7b) are provided, which are interchangeable with one another by means of a magnification changer, and whose magnification scales are reciprocal to one another.

6. Device according to one of claims 1 to 5, characterized in that, in order to alter the vertical magnification, a vertical deflecting device (8) may be appropriately actuated.

7. Device according to one of claims 1 to 6, characterized in that, for fine focusing or for displacement of the focal plane and for refractive compensation between a decoupling element (13) which separates the illuminating light (14) and the reflected light (15), and the scanner device, there is provided an optical system (1, 2, 4) which forms an intermediate image of a pupil plane P'' conjugate with the pupil of the eye to be investigated, and which includes at least one interchangeable and/or displaceable optical component (1).

8. Device according to claim 7, characterized in that the optical system has two stationary imaging optical elements (2, 4).

9. Device according to claim 8, characterised in that the stationary optical elements (2, 4) form an afocal system.

10. Device according to one of claims 7 to 9, characterised in that a lens (1) is disposed so as to be interchangeable, on a lens wheel (1''), in the beam path between the scanning system (5) and the decoupling element (13) .

11. Device according to one of claims 7 to 10, characterised in that the optical system has in addition at least two mirrors (3a, 3b) which respectively deflect the beam path in one plane through 90°, and are displaceable in common in order to alter the optical path length.

12. Device according to one of claims 7 to 11, characterised in that the lens wheel (1'') has a position in which no additional lens (1) can be brought into the beam path.

## Revendications

1. Dispositif pour la production de l'image d'un objet, et, destiné, en particulier, à l'observation des parties arrière de l'oeil, ce dispositif comportant une source lumineuse d'éclairage dont la lumière peut être focalisée sur l'objet,
un dispositif de balayage (5, 8) qui produit un déplacement de balayage du rayon lumineux de la source lumineuse d'éclairage sur l'objet et qui présente des éléments optiques déviant les rayons, ainsi que des éléments optiques focalisant les rayons,
un dispositif de détection qui reçoit la lumière réfléchie sur l'objet,
et un ensemble d'exploitation et de synchronisation qui produit une image de l'objet à partir du signal de sortie, séquentiel dans le temps, du dispositif de détection.
dispositif caractérisé en ce que pour la modification de l'angle de déviation horizontale, un dispositif composé d'au moins deux éléments optiques (6a, 60, 7a, 7b), focalisateurs et producteurs d'image, ainsi que des moyens de mise en place de chacun de ces éléments optiques focalisateurs sont prévus dans le chemin des rayons, entre les éléments optiques (5, 8) déviant les rayons.

2. Dispositif selon la revendication 1, caractérisé en ce que les éléments optiques de formation d'image par focalisation des rayons sont des miroirs.

3. Dispositif selon la revendication 1 ou 2, caractérisé en ce que le dispositif présente deux miroirs (6a, 6b, 7a, 7b) ou deux lentilles qui constituent un système afocal.

4. Dispositif selon la revendication 3, caractérisé en ce que les éléments optiques focalisants sont des miroirs de Mangin.

5. Dispositif selon l'une des revendications 1 à 4, caractérisé en ce que deux jeux d'éléments optiques focalisants (6a, 6b, 7a, 7b) sont prévus, lesquels peuvent être remplacés l'un par l'autre au moyen d'un dispositif de changement de grossissement, et dont les échelles de grossissement sont réciproques ou inverses l'une par rapport à l'autre.

6. Dispositif selon l'une des revendications 1 à 5, caractérisé en ce qu'un dispositif de déviation verticale (8) peut être commandé de façon adéquate en vue de la modification du grossissement dans le sens vertical.

7. Dispositif selon l'une des revendications 1 à 6, caractérisé en ce que pour le réglage de la mise au point ou pour le déplacement du plan de mise au point ainsi que pour la compensation de la réfraction, on prévoit, entre un élément de séparation (13) qui sépare la lumière d'éclairage (14) de la lumière réfléchie (15) et le dispositif de balayage (5), un système optique (1, 2, 4) qui réalise une focalisation intermédiaire dans un plan de pupille P'' conjugué à la pupille de l'oeil qu'il s'agit d'examiner, et qui comporte au moins un composant optique (1) remplaçable et/ou déplaçable.

8. Dispositif selon la revendication 7, caractérisé en ce que le système optique présente deux éléments optiques (2, 4) formateurs d'une image par focalisation et disposés de façon fixe.

9. Dispositif selon la revendication 8, caractérisé en ce que les éléments optiques fixes constituent un système afocal.

10. Dispositif selon l'une des revendications 7 à 9, caractérisé en ce qu'une lentille (1) est disposée, de façon à pouvoir être remplacée, sur un disque (1'') à lentilles, dans le chemin des rayons entre le système de balayage (5) et l'élément de séparation (13).

11. Dispositif selon l'une des revendications 7 à 10, caractérisé en ce que le système optique présente en plus au moins deux miroirs (3a, 3b) qui dévient d'au moins 90° le chemin des rayons dans un plan et qui peuvent être déplacés ensemble en vue de modifier la longueur du chemin optique.

12. Dispositif selon l'une des revendications 7 à 11, caractérisé en ce que le disque (1'') à lentilles présente une position dans laquelle aucune lentille supplémentaire (1) ne peut être mise en place sur le chemin des rayons.
